(19) Europäisches Patentamt
European Patent Office
Office européen des brevets

(11) Numéro de publication : **0 038 731**
**B1**

(12) **FASCICULE DE BREVET EUROPÉEN**

(45) Date de publication du fascicule du brevet : **26.10.83**

(21) Numéro de dépôt : **81400524.5**

(22) Date de dépôt : **01.04.81**

(51) Int. Cl.[3] : **C 07 D513/04**, A 61 K 31/425, A 61 K 31/505 // (C07D513/04, 277/00, 235/00),(C07D513/04, 277/00, 239/00)

(54) **Dérivés de thiazole, leur préparation et leur application en thérapeutique.**

(30) Priorité : **08.04.80 FR 8007845**

(43) Date de publication de la demande : **28.10.81 Bulletin 81/43**

(45) Mention de la délivrance du brevet : **26.10.83 Bulletin 83/43**

(84) Etats contractants désignés : **AT BE CH DE FR GB IT LI LU NL SE**

(56) Documents cités : **FR A 2 014 048**

**Le dossier contient des informations techniques présentées postérieurement au dépôt de la demande et ne figurant pas dans le présent fascicule.**

(73) Titulaire : **SYNTHELABO**
**58, rue de la Glacière**
**F-75621 Paris Cedex 13 (FR)**

(72) Inventeur : **Bigg, Dennis**
**5, Parc de Diane**
**F-78350 Jouy en Josas (FR)**

(74) Mandataire : **Thouret-Lemaitre, Elisabeth**
**Service Brevets - SYNTHELABO 58, rue de la Glacière**
**F-75621 Paris Cedex 13 (FR)**

**EP 0 038 731 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Dérivés de thiazole, leur préparation et leur application en thérapeutique

La présente invention concerne des dérivés de thiazole, leur préparation et leur application en thérapeutique.

Les composés de l'invention répondent à la formule (I)

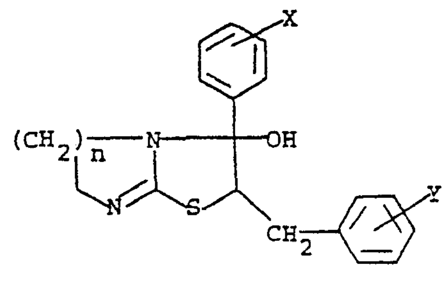

(I)

dans laquelle

n est 1 ou 2

X et Y représentent, indépendamment l'un de l'autre, un ou plusieurs substituants choisis dans le groupe constitué par les atomes d'hydrogène et d'halogène, les radicaux $CF_3$, alkyles et alcoxy droits ou ramifiés de 1 à 4 atomes de carbone.

Les sels que forment les composés (I) avec les acides pharmaceutiquement acceptables font partie de l'invention.

Les composés (I) peuvent également exister sous la forme ouverte suivante

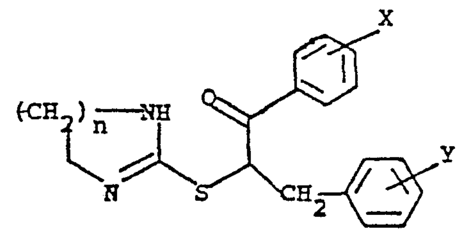

Les composés (I) comportent un centre d'asymétrie et peuvent exister sous la forme de racémates ou d'énantiomères qui font partie de l'invention.

Les composés préférés de l'invention sont ceux dans lesquels n est 1 et, dans ce cas, plus particulièrement, ceux dans lesquels chacun des radicaux X et Y, indépendamment l'un de l'autre, représente un ou plusieurs atomes d'hydrogène ou d'halogène ou radicaux $CF_3$, méthyle, tertiobutyle ou méthoxy et ceux dans lesquels n est 2 et, dans ce cas plus particulièrement, ceux dans lesquels chacun des radicaux X et Y, indépendamment l'un de l'autre, représente un ou plusieurs atomes d'hydrogène ou d'halogène ou radicaux méthyle, méthoxy, tertiobutyle, n-butoxy ou n-propoxy.

Des composés dérivés de thiazole portant un radical alkyle en position 2 ont déjà été décrits dans la demande de brevet FR-A-2 014 048 ; ces composés sont décrits comme possédant des propriétés pharmacodynamiques dans divers domaines.

Les composés de l'invention portant un radical benzyle en position 2 sont différents structuralement et pharmacologiquement de ces composés et possèdent des propriétés antidépressives très intéressantes.

Selon l'invention, on peut préparer les composés de l'invention selon les deux schémas réactionnels suivants :

a) 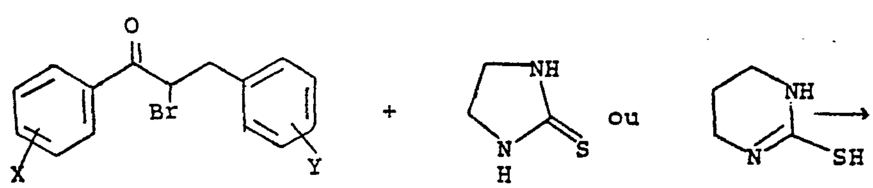

\+ ou

(I)

b) 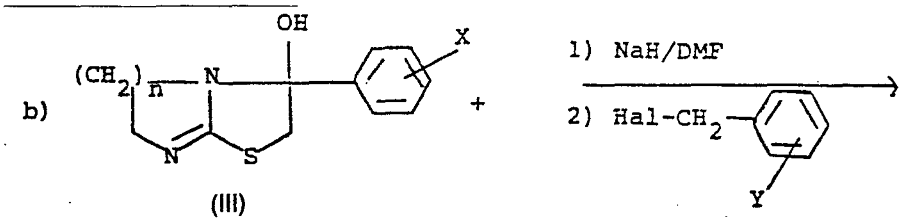


\+

(III)

(I)

Les produits obtenus par la voie a) peuvent être convertis en base par l'action d'une base appropriée (par exemple $Na_2CO_3$, $NaHCO_3$), de même que les produits obtenus par la voie b) peuvent être convertis en sels par des méthodes classiques.

La réaction entre la cétone (II) et l'éthylène-thiourée ou la tétrahydro-3,4,5,6 pyrimidine-thiol-2 est effectuée dans un solvant tel que l'acétone à la température ambiante.

La réaction entre le composé (III) et l'halogénure de benzyle substitué ou non par Y est effectuée dans un solvant tel que le diméthylformamide en présence d'hydrure de sodium, à la température ambiante.

Les cétones de départ (II) sont obtenues soit par la réaction classique du nitrile avec le réactif de Grignard, et ensuite l'hydrolyse, par exemple, selon la méthode décrite par W.J. Humphlett, M.J. Weiss et C.R. Hauser J. Am. Chem. Soc. *70,* 4020, 1948), soit par réaction du chlorure d'acide avec le réactif de Grignard dans du tétrahydrofuranne à − 78 °C, par exemple selon la méthode décrite par F. Sato, M. Inoue, K. Oguro et M. Sato, Tetrahedon Letters n° 44, pp. 4303-4306, (1979).

Les composés de départ (III) sont préparés à partir d'α-bromo-acétophénone substituée de manière correspondante et d'éthylène-thiourée ou de tétrahydro-3,4,5,6 pyrimidine-thiol-2, par exemple selon la méthode décrite par Sharpe and coll., Journal of Medicinal Chemistry, 1971, vol. 14, n° 10, p. 977-982.

Les exemples suivants illustrent l'invention.

Les analyses et les spectres IR et RMN confirment la structure des composés.

## Exemple 1

(Benzyl)-2 (trifluorométhyl-3 phényl)-3 tétrahydro-2,3,5,6 imidazo [2,1-b] thiazolol-3.

$$[n = 1,\ X = CF_3 - 3,\ Y = H]$$

Dans un erlenmeyer de 1 l, on introduit 2,24 g (0,022 mole) d'éthylène-thiourée dans 280 $cm^3$ d'acétone, et on ajoute 9,88 g (0,0277 mole) d'α-bromo-(trifluorométhyl-3)phényl-phénéthyl-cétone en solution dans 70 $cm^3$ d'acétone.

On agite pendant 20 h, filtre le précipité blanc apparu, lave à l'acétone, sèche et recristallise dans du méthanol contenant un peu de chloroforme. On obtient un solide blanc que l'on sèche sous vide, à la température ambiante, en présence de $P_2O_5$.

$$F_T = 169\text{-}170\ °C.$$

On obtient la base dans de l'eau, par addition de bicarbonate de sodium et extraction à l'aide d'acétate d'éthyle. On lave avec de l'eau et sèche. On évapore sous vide à 40 °C et reprend avec de l'éther. Le produit obtenu est recristallisé dans de l'acétone. On obtient un solide blanc.

$$F_T = 161\text{-}163\ °C.$$

## Exemple 2

(Fluoro-4 benzyl)-2 phényl-3 tétrahydro-2,3,5,6 imidazo [2,1-b] thiazolol-3.

$$[n = 1,\ X = H,\ Y = F - 4]$$

A 1,6 g (0,016 mole) d'éthylène-thiourée en solution dans 220 $cm^3$ d'acétone, on ajoute 9,85 g (0,032 mole) d'α-bromo-(fluoro-4 phénéthyl)-phényl-cétone en solution dans 80 $cm^3$ d'acétone. On agite pendant 20 h, puis filtre le précipité blanc apparu, le rince et le sèche. On obtient le bromhydrate du composé cherché.

$$F_T = 177\text{-}178\ °C.$$

On reprend ce composé dans un mélange d'eau et de chloroforme et on libère la base par addition de $Na_2CO_3$. On extrait avec du chloroforme, lave à l'eau, sèche et concentre. On reprend avec de l'éther, on obtient un solide blanc.

$$F_T = 169°\text{-}170\ °C.$$

## Exemple 3

Benzyl-2 (chloro-4 phényl)-3 tétrahydro-2,3,5,6 imidazo [2,1-b] thiazolol-3.

$$[n = 1,\ X = Cl - 4,\ Y = H]$$

Dans un ballon de 2 l, on introduit sous argon, 6,3 g (0,145 mole) d'hydrure de sodium à 56 %, lavé à

l'éther de pétrole. On ajoute 22,0 g (0,0865 mole) de (chloro-4 phényl)-3 tétrahydro-2,3,5,6 imidazo [2,1-b] thiazolol-3 en solution dans 300 $cm^3$ de DMF. On agite pendant 1 h, puis ajoute goutte à goutte 17,3 g (0,101 mole) de bromure de benzyle en solution dans 20 $cm^3$ de DMF. Après 1 h d'agitation, il se forme un précipité. On ajoute 400 $cm^3$ d'eau et 500 $cm^3$ d'éther. On filtre un solide blanc que l'on lave à l'acétate d'éthyle, puis à l'éther. On le recristallise dans un mélange $CHCl_3/CH_3OH$/Noir de carbone. On obtient un solide blanc que l'on sèche à 60 °C, sur $P_2O_5$, sous vide.

$F_T$ = 172-173,5 °C.

Exemple 4

(Méthyl-2 benzyl)-2 phényl-3 tétrahydro-2,3,5,6 imidazo [2,1-b] thiazolol-3.

[n = 1, X = H, Y = $CH_3$ − 2]

Dans un ballon de 2 l, on introduit, sous argon, 7,0 g (0,145 mole) d'hydrure de sodium à 50 %, lavé à l'éther de pétrole. On ajoute 19,06 g (0,0865 mole) de phényl-3 tétrahydro-2,3,5,6 imidazo [2,1-b] thiazolol-3 en solution dans 300 $cm^3$ de DMF. On agite pendant 1 h, puis ajoute goutte à goutte 18,7 g (0,101 mole) d'α-bromo-o-xylène en solution dans 30 $cm^3$ de DMF. Après 2 h, on ajoute au mélange réactionnel 400 $cm^3$ d'eau et 500 $cm^3$ d'éther. Il se forme un solide. On le filtre, le rince à l'eau, à l'acétone, puis à l'éther. On obtient un solide beige que l'on recristallise dans un mélange $CHCl_3/CH_3OH$/Noir de carbone. On obtient un solide blanc que l'on sèche sur $P_2O_5$, à la température ambiante, sous vide.

$F_T$ = 199-201 °C.

Exemple 5

Benzyl-2 (dichloro-2,4 phényl)-3 tétrahydro-2,3,6,7 5H-thiazolo [3,2-a] pyrimidinol-3.

[n = 2, X = 2,4 − $Cl_2$, Y = H]

Dans un erlenmeyer de 2 l, on introduit 12,3 g (0,034 mole) d'α-bromo-(dichloro-2,4 phényl)-phénéthyl-cétone en solution dans 50 $cm^3$ d'acétone dans une solution de 3,72 g (0,032 mole) de tétrahydro-3,4,5,6 pyrimidinethiol-2 dans 1 000 $cm^3$ d'acétone. On agite pendant 24 h. On obtient un solide blanc que l'on filtre, rince et sèche.

$F_T$ = 241-242 °C.

On libère la base de son sel dans un mélange d'eau et de chloroforme par addition de carbonate de sodium jusqu'à pH = 9. On extrait le mélange, laisse décanter, lave à l'eau, sèche et concentre le mélange réactionnel. On reprend l'huile obtenue dans de l'éther. Il se forme un précipité blanc que l'on sèche sous vide, à 60 °C, sur $P_2O_5$.

$F_T$ = 178-179 °C.

Dans le tableau suivant sont représentés les composés de l'invention préparés à titre d'exemples.

Tableau

| Composé | n | X | Y | $F_T$(°C) | |
|---|---|---|---|---|---|
| 1 | 1 | 4-Cl | H | 165-166<br>172-173,5 | chlorhydrate<br>base |
| 2 | 1 | 4-$CH_3O$ | H | 177-178 | base |
| 3 | 1 | H | H | 176-177 | base |
| 4 | 1 | 3,4-$Cl_2$ | H | 183-184<br>144-145 | base<br>méthane sulfonate |
| 5 | 1 | 4-Cl | 4-Cl | 171-172 | base |

(Fortsetzung)

| Composé | n | X | Y | | $F_T$(°C) |
|---|---|---|---|---|---|
| 6 | 1 | $4\text{-}CH_3$ | H | 164-164,5 | base |
| 7 | 1 | $3\text{-}CF_3$ | H | 161-163 | base |
| 8 | 1 | 2-Cl | H | 173,5-174,5<br>132,5-134 | bromhydrate<br>base |
| 9 | 1 | H | $2\text{-}CH_3$ | 199-201 | base |
| 10 | 1 | 3-Cl | H | 176-177 | base |
| 11 | 1 | 4-Br | H | 169-170 | base |
| 12 | 1 | $2,4\text{-}Cl_2$ | H | 159-160 | base |
| 13 | 1 | 3-Br | H | 174-175 | base |
| 14 | 1 | H | 4-F | 169-170 | base |
| 15 | 1 | $4\text{-t} \cdot C_4H_9$ | H | 192-193 | base |
| 16 | 2 | $2\text{-n}C_3H_7O$ | H | 167-168 | base |
| 17 | 2 | 2-Cl | H | 205-206 | bromhydrate |
| 18 | 2 | $2,4\text{-}Cl_2$ | H | 178-179 | base |
| 19 | 2 | 4-Br | H | 175,5-177 | base |
| 20 | 2 | 3-Br | H | 166-167 | base |
| 21 | 2 | H | 4-F | 128-129 | base |
| 22 | 2 | $2\text{-}CH_3$ | $2\text{-}CH_3O$ | 165-166 | base |
| 23 | 2 | 3-Cl | H | 148-150 | base |
| 24 | 2 | $4\text{-t} \cdot C_4H_9$ | H | 177-178 | base |
| 25 | 2 | $4\text{-n}C_4H_9O$ | H | 118-120 | base |

Les composés de l'invention (I) ont été soumis à des essais pharmacologiques qui ont montré leur activité antidépressive.

La toxicité des composés a été déterminée chez la souris par voie i.p. La DL 50 varie de 50 à plus de 1 000 mg/kg.

L'activité antidépressive a été déterminée selon le test de l'antagonisme vis-à-vis de la ptose réserpinique (Gouret C. et al., J. Pharmacol. (Paris) *8*, 333-350 (1977).

Les souris (mâles, CD1 Charles River, France, 18-22 g) reçoivent simultanément les produits à étudier ou le solvant (*voie i.p.*), et la réserpine (4 mg/kg, *voie s.c.*).

Soixante minutes plus tard, le degré de ptose palpébrale est estimé au moyen d'une échelle de cotation (0 à 4), pour chaque souris.

A chaque dose, la moyenne de cotation et le pourcentage de variation par rapport au lot contrôle, sont calculés.

Pour chaque produit, la DA 50, ou dose qui diminue de 50 % le score moyen de ptose par rapport aux contrôles, est déterminée graphiquement.

La DA 50 varie de 0,2 à 10 mg/kg par voie i.p.

Par ailleurs on a étudié l'action des drogues sur les pointes ponto-géniculo-occipitales lors du syndrome réserpinique chez le rat curarisé, afin de mettre en évidence les propriétés anti-dépressives des composés.

Chez le chat, on enregistre une activité spécifique au niveau du pont, du noyau genouillé latéral et du

cortex occipital qui fut appelée pointes ponto-géniculo-occipitales (P.G.O.)

Cette activité spontanée, (pointes P.G.O.), apparaissant lors du cycle veille-sommeil, peut être induite par la réserpine, agent pharmacologique diminuant le taux des monoamines cérébrales.

Cette activité électroencéphalographique, modulée par des mécanismes neuronaux sous le contrôle de neurotransmetteurs synaptiques, constitue donc un test pharmacologique pour l'étude de l'action centrale des drogues.

Toutes les expériences sont réalisées de manière aiguë sur des chats des deux sexes de 2 à 3 kg.

Avant le début de la préparation chirurgicale effectuée sous narcose à l'éther, l'animal reçoit une injection intra-péritonale de 0,75 mg/kg i.p. de réserpine.

Le chat est intubé pour permettre une ventilation artificielle.

Un anesthésique local (xylocaïne à 2 %) est injecté au niveau des points de pression et des incisions.

Des canules sont placées au niveau de la veine fémorale, pour l'injection des produits et l'infusion d'un agent curarisant, la gallamine triéthiodide (Flaxédil®), et de l'artère fémorale pour l'enregistrement de la pression artérielle.

Des électrodes monopolaires sont vissées au niveau cortical et des électrodes bipolaires coaxiales sont placées stéréotaciquement au niveau des noyaux genouillés latéraux.

L'animal curarisé et ventilé artificiellement est maintenu à température constante tout au long de l'expérience.

L'électroencéphalogramme, l'électrocardiogramme et la pression artérielle sont enregistrés à l'aide d'un polygraphe : Grass modèle 79 D.

Des doses croissantes (0,1 ; 0,3 ; 1 ; 10 ; et 30 mg/kg) des produits à étudier sont injectées par voie intraveineuse toutes les 30 minutes, 4 h 30 après l'administration de la réserpine.

Le nombre des pointes P.G.O. est comptabilisé automatiquement par périodes de 10 minutes et exprimé en pourcentage par périodes de 30 minutes en prenant la valeur obtenue lors des 30 minutes de contrôle comme référence (100 %).

La dose efficace réduisant de 50 % (DE 50) le nombre des pointes P.G.O. est calculée à l'aide d'une courbe de régression semi-logarithmique.

Les résultats sont les suivants : la DE 50 va de 0,2 à 3 mg/kg.

Les résultats pharmacologiques montrent que les composés de l'invention peuvent être utiles pour le traitement de la dépression.

Les composés de l'invention peuvent être présentés sous toute forme appropriée pour l'administration par voie orale ou parentérale, par exemple sous la forme de comprimés, dragées, gélules, solutions buvables ou injectables, etc... en association avec tout excipient approprié.

La posologie quotidienne peut aller de 5 à 200 mg.

**Revendications** (pour les Etats Contractants : BE, CH, DE, FR, GB, IT, LI, LU, NL, SE)

1. Dérivés de thiazole, sous la forme de racémates ou d'énantiomères, répondant à la formule (I)

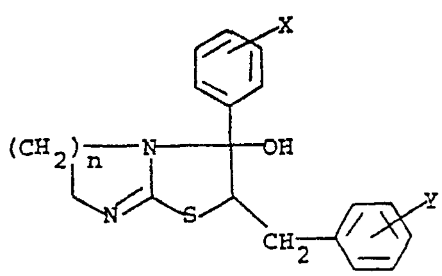

(I)

dans laquelle

n est 1 ou 2,

X et Y représentent, indépendamment l'un de l'autre, un ou plusieurs substituants choisis dans le groupe constitué par les atomes d'hydrogène ou d'halogène, les radicaux $CF_3$, alkyles et alcoxy droits ou ramifiés de 1 à 4 atomes de carbone, ainsi que leurs sels d'addition aux acides pharmaceutiquement acceptables.

2. Dérivés de thiazole selon la revendication 1, dans lesquels n est 1.

3. Dérivés de thiazole selon la revendication 1, dans lesquels n est 2.

4. Dérivés de thiazole selon la revendication 2, dans lesquels X et Y représentent indépendamment l'un de l'autre, un ou plusieurs atomes d'hydrogène ou d'halogène ou radicaux $CF_3$, méthyle, tertiobutyle ou méthoxy.

5. Dérivés de thiazole selon la revendication 3, dans lesquels X et Y représentent, indépendamment l'un de l'autre, un ou plusieurs atomes d'hydrogène ou d'halogène ou radicaux méthyle, méthoxy, tertiobutyle, n-butoxy ou n-propoxy.

6. Le benzyl-2 (dichloro-3,4 phényl)-3 tétrahydro-2,3,5,6 imidazo [2,1-b] thiazolol-3 et ses sels.

7. Le benzyl-2 (bromo-4 phényl)-3 tétrahydro-2,3,5,6 imidazo [2,1-b] thiazolol-3 et ses sels.

8. Procédé de préparation des composés selon la revendication 1, procédé caractérisé en ce que l'on fait réagir une cétone de formule (II)

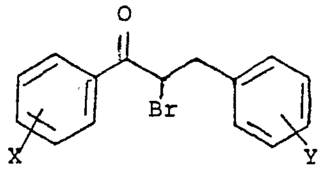

(II)

X et Y ayant les significations données dans la revendication 1, avec de l'éthylène-thiourée ou du tétrahydro-3,4,5,6 pyrimidine-thiol-2.

9. Procédé de préparation des composés selon la revendication 1, procédé caractérisé en ce que l'on fait réagir un composé de formule (III)

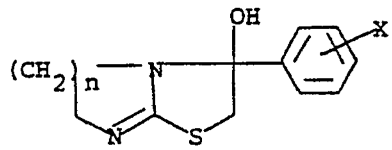

(III)

avec l'hydrure de sodium et ensuite l'halogénure de benzyle de formule

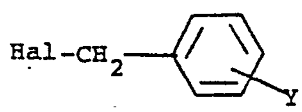

X et Y ayant les significations données dans la revendication 1.

10. Médicament caractérisé en ce qu'il contient un composé tel que spécifié dans l'une quelconque des revendications 1 à 7.

**Revendication** (pour l'Etat Contractant AT)

Procédé de préparation de dérivés de thiazole, sous la forme de racémates ou d'énantiomères, répondant à la formule (I)

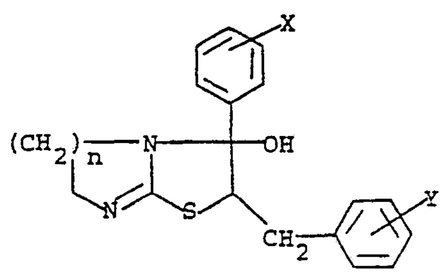

(I)

dans laquelle

n est 1 ou 2,

X et Y représentent, indépendamment l'un de l'autre, un ou plusieurs substituants choisis dans le groupe constitué par les atomes d'hydrogène ou d'halogène, les radicaux $CF_3$, alkyles et alcoxy droits ou ramifiés de 1 à 4 atomes de carbone, ainsi que de leurs sels d'addition aux acides pharmaceutiquement acceptables, procédé caractérisé en ce que l'on fait réagir une cétone de formule (II) .

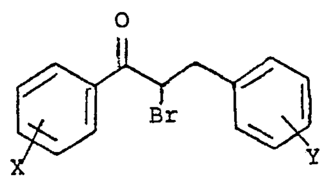

(II)

avec de l'éthylène-thiourée ou du tétrahydro-3,4,5,6 pyridine-thiol-2.

**Claims** (for the Contracting States : BE, CH, DE, FR, GB, IT, LI, LU, NL, SE)

1. Thiazole derivatives, in the form of racemates or enantiomers, corresponding to the formula (I)

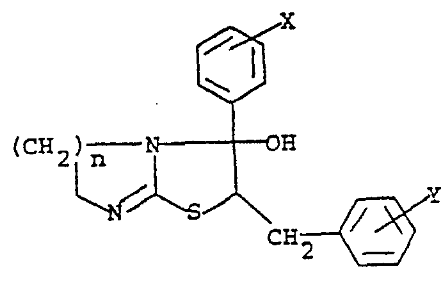

(I)

in which

n is 1 or 2 and

X and Y independently of one another represent one or more substituents chosen from the group comprising hydrogen atoms and halogen atoms, $CF_3$ radicals and linear or branched alkyl and alkoxy radicals having 1 to 4 carbon atoms, and also their addition salts with pharmaceutically acceptable acids.

2. Thiazole derivatives according to Claim 1, in which n is 1.

3. Thiazole derivatives according to Claim 1, in which n is 2.

4. Thiazole derivatives according to Claim 2, in which X and Y independently of one another represent one or more hydrogen or halogen atoms or $CF_3$, methyl, tert.-butyl or methoxy radicals.

5. Thiazole derivatives according to Claim 3, in which X and Y independently of one another represent one or more hydrogen or halogen atoms or methyl, methoxy, tert.-butyl, n-butoxy or n-propoxy radicals.

6. 2-Benzyl-3-(3,4-dichlorophenyl)-2,3,5,6-tetrahydroimidazo [2,1-b] thiazol-3-ol and its salts.

7. 2-Benzyl-3-(4-bromophenyl)-2,3,5,6-tetrahydro-imidazo [2,1-b] thiazol-3-ol and its salts.

8. Process for the preparation of the compounds according to Claim 1, which process is characterised in that a ketone of the formula (II)

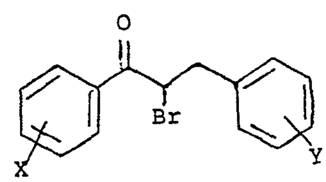

(II)

in which X and Y have the meanings given in Claim 1, is reacted with ethylene-thiourea or 3,4,5,6-tetrahydropyrimidine-2-thiol.

9. Process for the preparation of the compounds according to Claim 1, which process is characterised in that a compound of the formula (III)

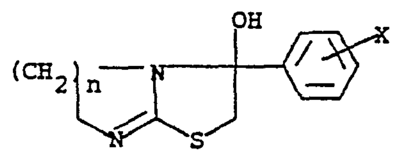

(III)

is reacted with sodium hydride and then with the benzyl halide of the formula

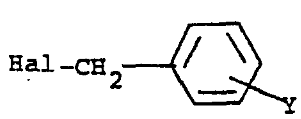

in which X and Y have the meanings given in Claim 1.

10. Medicament, characterised in that it contains a compound as specified in any of Claims 1 to 7.

**Claim** (for the Contracting State AT)

Process for the preparation of thiazole derivatives, in the form of racemates or enantiomers, corresponding to the formula (I)

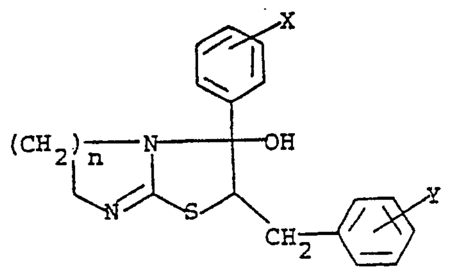

(I)

in which

n is 1 or 2 and

X and Y independently of one another represent one or more substituents chosen from the group comprising hydrogen atoms and halogen atoms, $CF_3$ radicals and linear or branched alkyl and alkoxy radicals having 1 to 4 carbon atoms, and also their addition salts with pharmaceutically acceptable acids which process is characterised in that a compound of the formula (II)

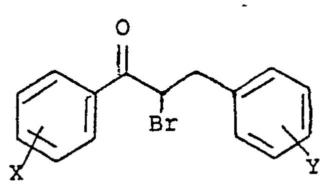

(II)

is reacted with ethylene-thiourea or 3,4,5,6-tetrahydropyrimidine-2-thiol.

**Ansprüche** (für die Vertragsstaaten : BE, CH, DE, FR, GB, IT, LI, LU, NL, SE)

1. Thiazolderivate in Form ihrer Racemate oder Enantiomeren, die der Formel (I)

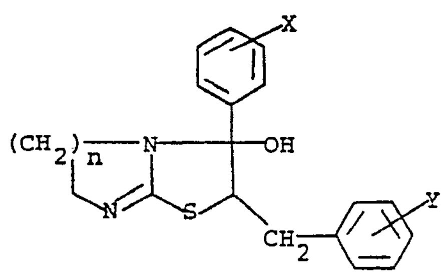

(I)

entsprechen, in welcher

n für 1 oder 2 steht und

X und Y unabhängig voneinander einen oder mehrere Substituenten, ausgewählt aus der Gruppe bestehend aus den Wasserstoff- oder Halogenatomen, dem Rest $CF_3$ sowie den geradkettigen oder verzweigten Alkyl- und Alkoxygruppen mit 1 bis 4 Kohlenstoffatomen, bedeuten, sowie deren Säureadditionssalze von pharmazeutisch annehmbaren Säuren.

2. Thiazolderivate nach Anspruch 1, bei welchen n für 1 steht.

3. Thiazolderivate nach Anspruch 1, bei welchen n für 2 steht.

4. Thiazolderivate nach Anspruch 2, bei welchen X und Y unabhängig voneinander ein oder mehrere Wasserstoff- oder Halogenatome oder die Reste $CF_3$, Methyl, Tertiobutyl oder Methoxy bedeuten.

5. Thiazolderivate nach Anspruch 3, bei welchen X und Y unabhängig voneinander ein oder mehrere Wasserstoff- oder Halogenatome oder die Reste Methyl, Methoxy, Tertiobutyl, n-Butyl oder n-Propoxy bedeuten.

6. Das 2-Benzyl-3-(3,4-dichlorphenyl)-2,3,5,6-tetrahydroimidazo [2,1-b]-thiazolol-3 und seine Salze.

7. Das 2-Benzyl-3-(4-bromphenyl)-2,3,5,6-tetrahydro-imidazo [2,1-b]-thiazolol-3 und seine Salze.

8. Verfahren zur Herstellung von Verbindungen nach Anspruch 1, dadurch gekennzeichnet, daß man ein Keton der Formel (II)

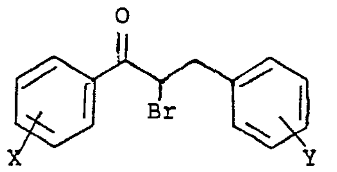

(II)

in der X und Y die im Anspruch 1 genannten Bedeutungen haben, mit Äthylenthioharnstoff oder 3,4,5,6-Tetrahydropyrimidinthiol-2 umsetzt.

9. Verfahren zur Herstellung von Verbindungen nach Anspruch 1, dadurch gekennzeichnet, daß man eine Verbindung der Formel (III)

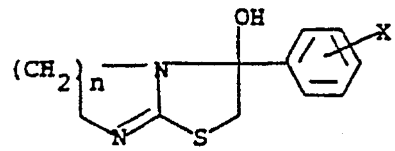

(III)

mit Natriumhydrid und anschließend mit dem Benzylhalogenid der Formel

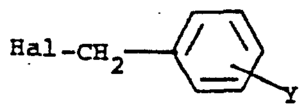

in welcher X und Y die im Anspruch 1 angegebenen Bedeutungen haben, umsetzt.

10. Arzneimittel, dadurch gekennzeichnet, daß es eine der in den Ansprüchen 1 bis 7 dargelegten Verbindungen enthält.

**Anspruch** (für den Vertragsstaat AT)

Verfahren zur Herstellung von Thiazolderivaten in Form ihrer Racemate oder Enantiomeren, die der Formel (I)

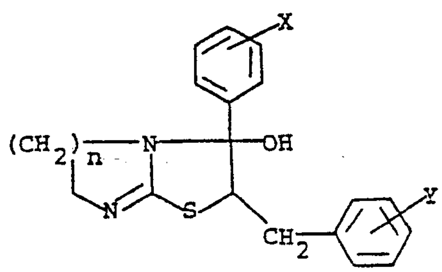

(I)

entsprechen, in welchen

n für 1 oder 2 steht und

X und Y unabhängig voneinander einen oder mehrere Subsituenten, ausgewählt aus der Gruppe bestehend aus den Wasserstoff- oder Halogenatomen, dem Rest $CF_3$ sowie den geradkettigen oder verzweigten Alkyl- und Alkoxygruppen mit 1 bis 4 Kohlenstoffatomen, bedeuten, sowie deren Säureadditionssalzen von pharmazeutisch annehmbaren Säuren, dadurch gekennzeichnet, daß man ein Keton der Formel (II)

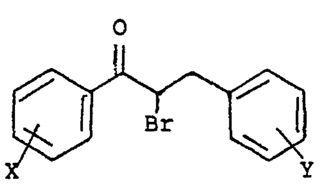

(II)

mit Äthylenthioharnstoff oder 3,4,5,6-Tetrahydropyrimidinthiol-2 umsetzt.